# EUROPEAN PATENT SPECIFICATION

(11) **EP 4 065 185 B1**
(45) Date of publication and mention of the grant of the patent: **15.07.2026**
(21) Application number: 20824895.5
(22) Date of filing: 27.11.2020
(51) Int. Cl.: A61L 27/36

(54) **AN ACELLULAR NERVE GRAFT**
AZELLULÄRES NERVENTRANSPLANTAT
GREFFE DE NERF ACELLULAIRE

(30) Priority: 29.11.2019 EP 19212601
(43) Date of publication of application: 05.10.2022
(73) Proprietor: The Provost, Fellows, Scholars and other Members of Board of Trinity College Dublin, Dublin 2 (IE); Royal College of Surgeons in Ireland, Dublin 2 (IE)
(72) Inventor: BUCKLEY, Conor, Greystones, Co. Wicklow (IE); ZILIC, Leyla, Manchester M23 0PU (GB); O'BRIEN, Fergal, Dublin, 18 (IE)
(74) Representative: Purdylucey Intellectual Property
(86) International application number: PCT/EP2020/083811
(87) International publication number: WO 2021/105488

(56) References cited:
- EP-A2- 2 255 833
- WO-A1-2014/207744
- LEYLA ZILIC ET AL: "Decellularisation and histological characterisation of porcine peripheral nerves : Decelluarisation of Peripheral Nerve", BIOTECHNOLOGY AND BIOENGINEERING, vol. 113, no. 9, 1 September 2016 (2016-09-01), Hoboken, USA, pages 2041 - 2053, XP055541212, ISSN: 0006-3592, DOI: 10.1002/bit.25964
- RUKMANI SRIDHARAN ET AL: "Decellularized grafts with axially aligned channels for peripheral nerve regeneration", JOURNAL OF THE MECHANICAL BEHAVIOR OF BIOMEDICAL MATERIALS, vol. 41, 1 January 2015 (2015-01-01), AMSTERDAM, NL, pages 124 - 135, XP055378765, ISSN: 1751-6161, DOI: 10.1016/j.jmbbm.2014.10.002

## Description

### Field of the Invention

The present invention relates to an acellular nerve graft, and methods for the production thereof. Also contemplated is the acellular nerve graft for use in the treatment of long nerve gap injuries in a subject.

### Background to the Invention

The nervous system is one of the most highly organised systems within the human body, integrating various bodily processes and reactions of the organism to its environment. It is subdivided into the central nervous system (CNS) and peripheral nervous system (PNS). The CNS is the larger system, including the brain and spinal cord. The PNS involves a vast network of nerves that are linked to the brain and the spinal cord receptors. The function of the PNS is to help process changes in the internal and external environment. Injuries to peripheral nerves are common and debilitating and may result in loss of motor function, sensory function, or both. In the United States over twenty million Americans suffer from peripheral nerve injury (Lundborg 2003) and over $150 billion is spent annually on treating these injuries (Taylor, Braza et al. 2008).

Previous studies have demonstrated that decellularised nerve extracellular matrix (ECM) can be used as a material to generate scaffolds for nerve regeneration, however producing an acellular scaffold for treatment of long peripheral nerve gap injuries has proved challenging. Furthermore, it is unclear if these scaffolds can be further enhanced by the addition of extra ECM components such as glycosaminoglycans (GAGs) to help stimulate the regeneration process.

Methods of preparing acellular peripheral nerve grafts are described in US14274156, US10672689, WOUS16042273, US14776765, US13776606, US13525753, US10624534, WOUS16031313, US10615623 and US10478198.

Buckley et al. (Sridharan, R., Reilly, R.B. and Buckley C.T. Decellularized grafts with axially aligned channels for peripheral nerve regeneration. Journal of the Mechanical Behaviour of Biomedical Materials 41: 124-135, 2015) describes short and narrow acellular peripheral nerve grafts formed by a method comprising a decellularization step comprising chemical and enzymatic washing of the section of nerve followed by a unidirectional freeze-drying step to impart porosity into the nerve tissue. The nerves employed in this study were small rat nerves, and the content of DNA in the decellularized nerves was about 257 +/- 36.9 ng/mg.

Zilic et al. (Zilic, L., Wilshaw, S.-P. and Haycock, J.W. (2016), Decellularisation and histological characterisation of porcine peripheral nerves. Biotechnol. Bioeng., 113: 2041-2053) describes the decellularisation of peripheral nerve grafts by a method comprising a freeze-thaw cycle, treatment with hypotonic buffer and hypotonic buffer containing SDS in the presence of Aprotinin and EDTA, washing in PBS, treatment with Benzonase, treatment with a hypertonic buffer, and sterilising with peracetic acid. The resulting acellular nerve grafts have a DNA content of about 37 ng/mg.

It is an object of the invention to overcome at least one of the above-referenced problems.

### Summary of the Invention

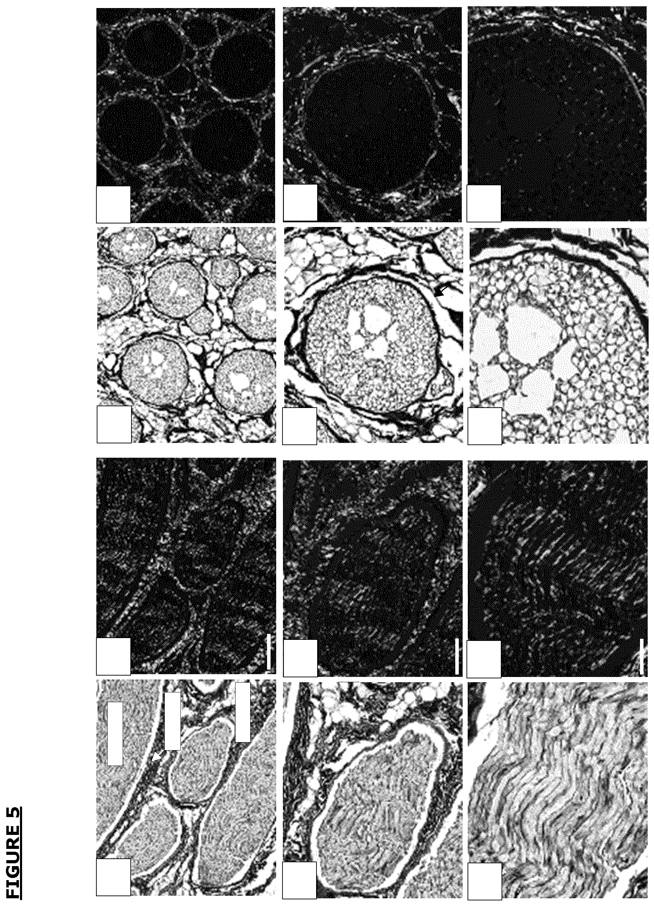

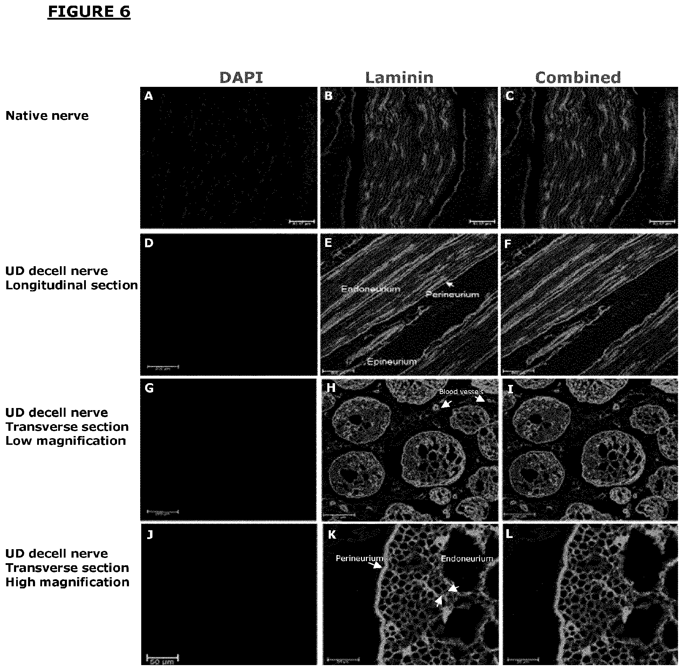

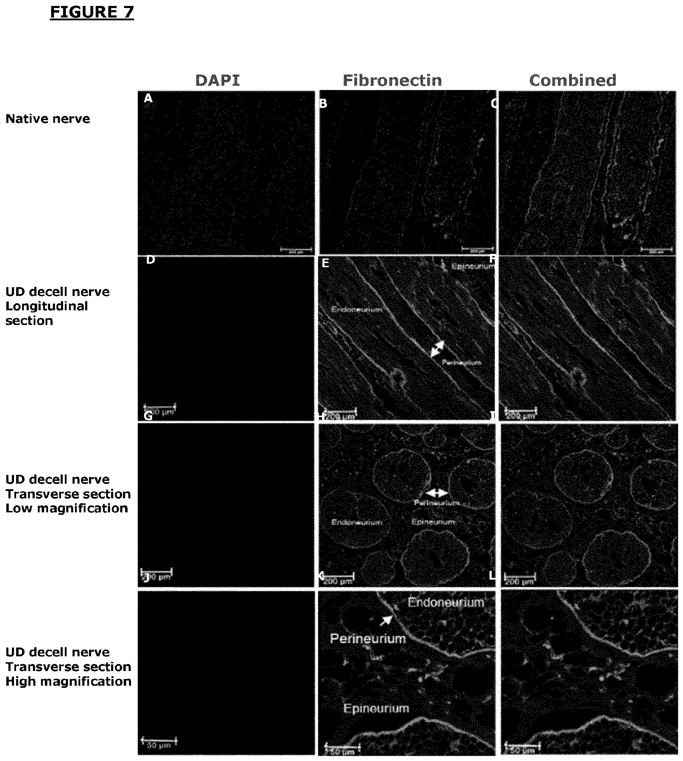

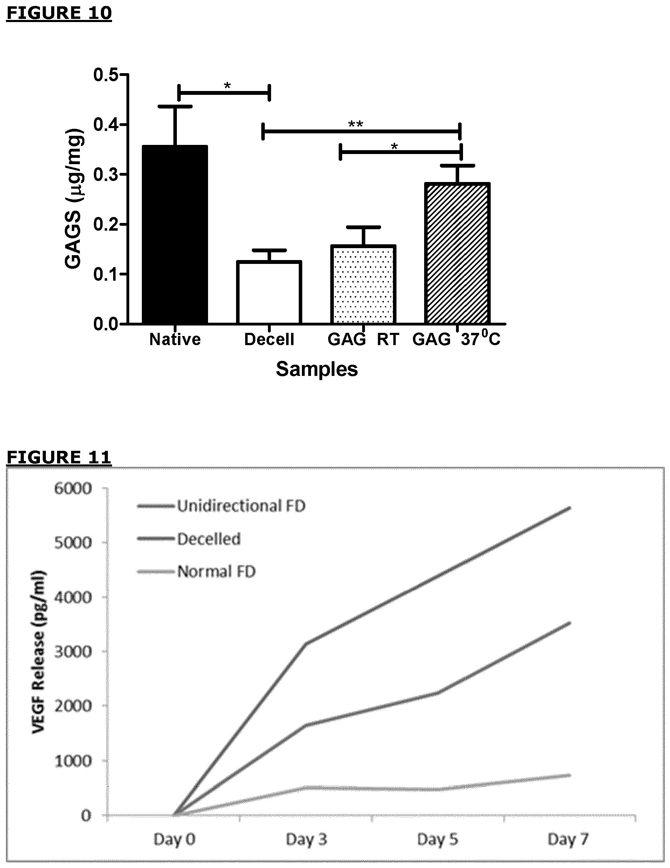

The Applicant has discovered that the methods of the prior art can be improved by performing an initial freeze-thaw treatment step, then performing unidirectional freeze-drying of the nerve tissue to introduce longitudinal pores into the section of nerve, and performing a chemical and enzymatic decellularization step on the *porous* section of nerve. In particular, and without being bound by theory, the Applicant has found that performing unidirectional freeze-drying prior to chemical/enzymatic treatment allows for greater penetration of the chemical and enzymatic decellularization agents into the section of nerve due to the presence of pores in the freeze-dried nerve tissue, resulting in greater decellularization of the nerve tissue (e.g. less than 70 or 60 ng/mg (dry weight) of DNA with the method of one embodiment of the invention, compared with 257 ng/mg with the prior art method). Data presented also shows retention of high levels of the ECM components laminin, fibronectin and collagen (Figs 6-8), retention of native nerve structure (Fig. 5), and a narrower distribution of pore sizes. In addition, the Applicant has discovered that an acellular nerve graft according to the invention exhibits enhanced VEGF release compared to acellular nerve grafts formed according to the prior art methods (Fig. 11). In a separate, but related, aspect, the Applicant has also provided an acellular nerve graft containing a high level of sulphated glycosaminoglycan (sGAG) (Fig. 10), and a method for the production thereof.

In a first aspect, the invention provides a method of producing an acellular nerve graft comprising the steps of:
providing a section of peripheral nerve;
primary treatment of the section of nerve comprising freezing and then thawing the section of peripheral nerve;
freeze-drying the section of nerve longitudinally and unidirectionally to introduce longitudinal pores into the section of peripheral nerve having an average pore size of at least 40 µm; and
decellularization of the section of nerve comprising contacting the freeze-dried section of nerve with detergent and enzymatic decellularization agents to provide the acellular nerve graft havign a DNA content of less than 70 ng/mg.

In any embodiment, the section of nerve is a section of peripheral nerve. The method of the invention has been found to be particularly suitable for longer sections of nerve, of the type used to repair long peripheral nerve gap injuries. In one embodiment, the section of nerve has a length of at least 40 mm, 50 mm, 60 mm, 70 mm, 80 mm, 90 mm, or 100 mm.

The method of the invention comprises freeze-drying the section of nerve longitudinally, whereby longitudinal pores are introduced into the tissue. The pores are unidirectional and generally co-axial with a longitudinal axis of the section of nerve. Unidirectional freeze-drying of sections of nerve is described in Buckley et al. In one embodiment, the longitudinal freeze-drying comprises freeze-drying the thawed section of nerve in an upright position in the freeze-drying chamber.

In any embodiment, the thawed section of nerve is placed into a tube formed in an insulating block, and freeze-dried within the insulating block. In one embodiment, the tube is an upright tube and the section of nerve is freeze-dried in an upright orientation. In one embodiment, the insulating block comprises ertalyte polymer.

In any embodiment, the primary treatment step comprises freezing the section of peripheral nerve to -70°C to -90°C, typically about 80°C. In one embodiment, the section of nerve is wrapped in filter paper during thawing.

In any embodiment, the freeze-drying conditions comprise a freezing stage at a freezing temperature, a primary drying stage at a first drying temperature that is less than 0°C and higher than the freezing temperature, and a secondary drying stage at a second drying temperature that is higher than the first drying temperature. In any embodiment, the freezing temperature is less than -10°C or -20°C, for example -20-40°C, preferably about - 30°C. In any embodiment, the first drying temperature is about -5 to -20°C, for example about -10°C. In any embodiment, the freezing stage lasts for about 30-90 minutes (ideally about 60 minutes when the freezing temperature is about -30°C). In any embodiment, the first drying stage lasts for about 12 to 36 hours (ideally about 24 hours when the first drying temperature is about -10°C). In any embodiment, the second drying stage lasts for about 1 to 10 hours (ideally about 5 hours when the second drying temperature is about 20°C).

Thus, in any embodiment, the freeze-drying conditions comprise a freezing stage at about - 30°C for about 1 hour, a primary drying stage at a temperature of about -10°C for about 24 hours, and a secondary drying stage at a temperature of about 20°C for about 5 hours.

In any embodiment, the method comprises a further step of contacting the acellular nerve graft with a solution of sulphated glycosaminoglycan (sGAG), for example chondroitin-6-sulphate, typically at elevated temperature, i.e. a temperature greater than room temperature, for example greater than 25°C, 30°C, 35°C or 40°C, for example 25-45°C, 25-40°C, 30-40°C.

In another aspect, the invention provides an acellular nerve graft obtained by or obtainable by a method of the invention.

Such an acellular nerve graft is prepared from native nerve tissue and comprises reduced DNA content (e.g. compared with the prior art methods), of less than 70, or 60 ng/mg.

The acellular nerve graft is prepared from native nerve tissue and comprises longitudinal pores having an average pore size of at least 40µm.

Preferably the acellular nerve graft prepared from native nerve tissue comprises a native nerve structure with retention of nerve fascicles and connective tissue layers, endoneurium, perineuriem and epineurium.

In another preferred embodiment, the acellular nerve graft prepared from native nerve tissue comprises a sulphated glycosaminoglycan (sGAG) content of greater than 0.21 µg/mg.

According to the claimed invention, the acellular nerve graft is an acellular peripheral nerve graft.

The acellular nerve graft has an average pore size of at least 40, 50, 60, 70, 80, 90 or 100 µm when determined by the method of determining average pore size as described below. The pore size may be up to 150 µm. The pore size may be up to 200 µm.

According to the claimed invention, the acellular nerve graft has an average pore size of at least 40 µm.

According to the claimed invention, the acellular nerve graft has a DNA content (dry weight) of less than 70, 65, 60, or 57 ng/mg.

In one embodiment, the acellular nerve graft has a native nerve structure with retention of nerve fascicles and connective tissue layers, endoneurium, perineuriem and epineurium.

In one embodiment, the acellular nerve graft has a sulphated glycosaminoglycan (sGAG) content of greater than 0.10 µg/mg, 0.15 µg/mg, or 0.21 µg/mg.

In one embodiment, the invention provides an acellular peripheral nerve graft characterised by comprising:
a length of at least 50 mm;
longitudinal pores having an average pore size of at least 40 m;
a DNA content of less than 70 ng/mg;
a native nerve structure with retention of nerve fascicles and connective tissue layers, endoneurium, perineuriem and epineurium; and
optionally, a sulphated glycosaminoglycan (sGAG) content of greater than 0.21 µg/mg.

Also described herein, but not part of the claimed invention, is the acellular peripheral nerve graft for use in a method of treating a nerve gap injury (especially long nerve gap injury) in a subject, comprising a step of implanting an acellular nerve graft of the invention into the subject to bridge the gap in the nerve.

In one embodiment, the acellular nerve graft is an allograft.

In one embodiment, the nerve gap injury is a peripheral nerve gap injury.

In one embodiment, the nerve gap injury comprises a long nerve injury (e.g.. a gap of greater than 30 mm), and wherein the acellular nerve graft has a length of greater than 30 mm.

Other aspects and preferred embodiments of the invention are defined and described in the other claims set out below.

### Brief Description of the Figures

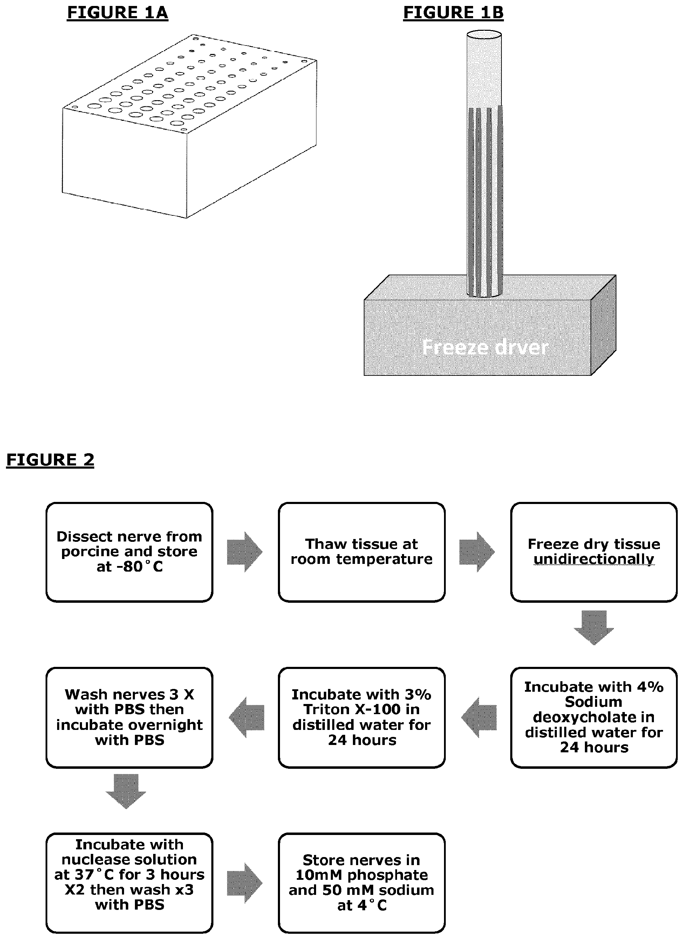

**Figure** 1: *Unidirectional freeze drying conditions.* Ertalyte polymer block used to place nerves in a longitudinal direction (A); Diagram of nerve in upright position to allow the formation of ice crystals in a longitudinal direction (in blue) to create unidirectional pores (B).
**Figure** 2: *Summary of optimised decellularisation process.* The optimised decellularisation process is comprised of freeze-thawing, unidirectional freeze drying, chemical and enzymatic washing steps
**Figure** 3: *Unidirectional freeze dried (UFD) porcine peripheral nerves.* Scanning electron microscopy images (SEM) of transverse nerve sections (A-B) and picrosirius red staining for collagen of longitudinal nerve sections under normal (C, E) and polarised light (D, F) shows how unidirectional freeze drying increases the porosity between the nerve fibres. Quantitative analysis demonstrates a significant increase in pore size within the unidirectional freeze dried nerves, with an average pore size of 45.8 µm when compared to native nerve which has an average pore size of 4.8 µm (G, H).

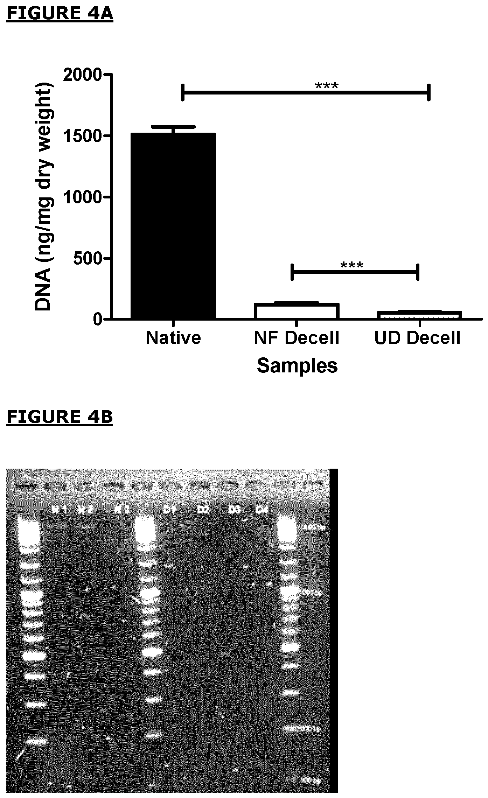

**Figure 4****:** *Unidirectional freeze drying aids the decellularisation process in the removal of cells from peripheral nerve tissue.* Acellular unidirectional freeze dried nerve (UD) show significantly less DNA (55 ng/mg) when compared to decellularised normal freeze dried nerve (NF) (121 ng/mg). The unidirectional freeze drying decellularisation process eliminates over 96% of DNA from the tissue when compared to native nerve (A). Gel electrophoresis (B) demonstrates that native porcine nerve tissue (N1-N3) contains DNA fragments which are over 3000 base pairs (bp) long; The remaining DNA fragments in the decellularised nerve tissue (D1-4) are less than 100 bp long. NF = normal freeze dried; UD = unidirectional freeze dried; N1-N3 = native porcine nerve tissue; D1-D4 = decellularised porcine nerve tissue.
**Figure 5****:** *Optimised decellularisation method retains the nerves native structure.* Histological staining of the acellular nerve tissue reveals the removal of cell nuclei and the retention of the nerve fascicles and connective tissue layers, the endoneurium, perineurium and epineurium. For light microscope images Red= collagen fibres, blue = myelin, purple/black = cell nuclei. For polarised light images Red = thick collagen fibres, green = thin collagen fibres
**Figure 6****:** *Representative histological images from the central region of native and acellular porcine peripheral nerves labelled using a monoclonal antibody against laminin.* Antibodies to laminin delineate a discrete ring around each Schwann cell/neuron unit in the endoneurium (H-I and K-L), the lamellar structure of the perineurium (E-F, H-I and K-L) and around blood vessels (H and I). Unstained round areas are spaces occupied by axon/myelin units as indicated by the arrows (K). Images (K and L) show retention of laminin within the endoneurium and around the perineurium of the fascicle following decellularisation. The ring-like appearance of the open tubes in the acellular nerves (C-D) suggests preservation of the basal lamina. The lack of DAPI staining in the acellular nerves (G and J) is due to the effective removal of cells during the decellularisation process. UD= unidirectional freeze dried.
**Figure** 7: *Representative histological images from the central region of native and acellular porcine peripheral nerves labelled using a monoclonal antibody against fibronectin.* In the acellular nerves antibodies to fibronectin delineates the lamellar structure of the perineurium (E, H, and K) and is detected sporadically within the endoneurium and surrounding epineurium (H, K). UD= unidirectional freeze dried.
Figure 8: *Collagen content of native and acellular nerve tissue determined by a hydroxyproline* assay *at 570 nm.* Native nerves have an average collagen content of 178.8 ± 13.79 µg/mg (n=9); acellular nerves have an average collagen content of 255.8 ± 26.89 µg/mg (n=9). There was a significant increase in collagen after the decellularisation process. This increase is due to the removal of other ECM components and decrease in mass of nerve segment. Data was analysed using a student's t-test and is presented as the mean ± 95 % C.I (p<0.0215*).
Figure 9: *Sulphated proteoglycan content of native and acellular nerve tissue determined by glycosaminoglycan (GAG) quantification* assay *at* 656 *nm.* There is a significant reduction in GAG content after the decellularisation process. Native nerves have an average GAG content of 2.89 ± 0.63 µg/mg (n=9); acellular nerves have an average GAG content of 0.37 ± 0.07 µg/mg (n=9). Data was analysed using a student's t-test and is presented as the mean ± 95 % C.I (p<0.0001**).
Figure 10: *Sulphated GAG content in acellular nerve tissue after the addition of chondroitin-6-sulphate.* There is a significant increase in GAG content after incubating the acellular nerves in chondroitin-6-sulphate solution at 37°C when compared to the decell tissue (processed acellular tissue). Native nerves have an average GAG content of 0.36± 0.08 µg/mg (n=9); acellular nerves 0.13 ± 0.02 µg/mg (n=9); acellular nerves incubated with chondroitin-6-sulphate at RT 0.16 ± 0.04 µg/mg (n=9); acellular nerves incubated with chondroitin-6-sulphate at 37°C 0.28 ± 0.04 µg/mg (n=9). Data was analysed using a student's t-test and is presented as the mean ± 95 % C.I (p<0.0023**). GAG = glycosaminoglycan; RT = room temperature.
Figure 11: *Unidirectional freeze-drying has beneficial effect on enhancing VEGF release compared to standard freeze-drying protocol.*
**Figure 12****:** In vitro assessment - effects of sterilisation on bioactivity. Three sterilisation groups; Peracetic acid; Ethylene Oxide; Gamma irradiation; Collagen gel control.
**Figure 13****:** In vitro assessment - effects of sterilisation on mechanical properties. (A) ETO sterilisation increases mechanical properties. (b) Gamma irradiation has no effect compared to native nerve.
**Figure 14****:** In-vivo assessment. Rat nerve gap injury model. 8 week study with (A)15 autografts with cuffs, and (B)15 decellularised nerve grafts (DNG).
**Figure 15****:** In vivo assessment - sensory stimulation testing: (A) autograft; (B) decellularized nerve graft v Autograft; (C) decellularized nerve graft; (D) table of comparison.
**Figure 16****:** In vivo assessment - response to electrical stimulation (A) compound nerve action potential (CNAP) for autograft (n=12) and decellularized graft (n=15); (B) compound muscle action potential (CMAP) for autograft (n=14) and decellularized graft (n=9). CNAP peak sized greater for decellularized graft and CNAP duration of conducted impulse greater for autograft. No significant differences between the autograft and decellularized nerve graft.
**Figure 17****:** In-vivo assessment - muscle mass (A) autograft (n=12) for treated and healthy and (B) DNG (n=14) for treated and healthy. (C) Comparison of mass of treated muscle tissue (DNG v Autograft)
**Figure 18****:** In-vivo assessment - macroscopic examination: (A and B) autograft and (C and D) Decellularised Nerve Graft. No "bottle neck" appearance in any of the grafts at mid-region. No evidence of neuroma formation observed at either suture line or along length. No apparent evidence of rejection or degeneration. Formation of abundant new ECM tissue comparable to autograft.
**Figure 19****:** In-vivo assessment - Histological assessment
**Figure 20****:** In-vivo assessment - Hjstological assessment. Neurofilament staining, examination of axonal sprouting in autograft and decellularized nerve graft.
**Figure** 21: In-vivo assessment - Hjstological assessment. S100-beta staining of localised schwann cell growth.
Figure 22: In-vivo assessment - Hjstological assessment. Laminin staining assesses basal lamina integrity.

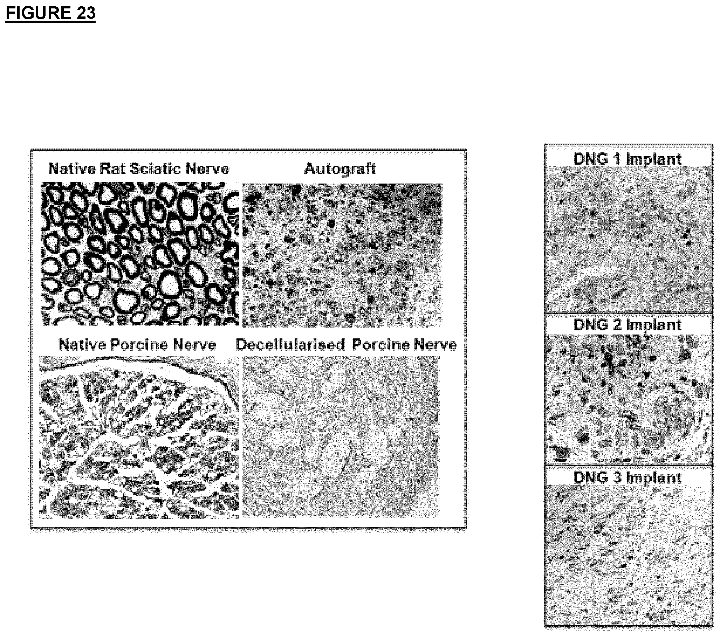

Figure 23: In-vivo assessment - Hjstological assessment. Toluidine blue staining analysis of myelinated and un-myelinated axons. Comparison between Native rat sciatic nerve, autograft, native porcine nerve, decellularised porcine nerve and DNG implant.

### Detailed Description of the Invention

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or *"an"* used in relation to an entity is to be read to refer to one or more of that entity. As such, the terms "a" (or *"an*"), *"one or more,"* and "at *least one"* are used interchangeably herein.

As used herein, the term *"comprise," or* variations thereof such as *"comprises"* or *"comprising,"* are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term *"disease"* is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, age, poisoning or nutritional deficiencies.

As used herein, the term *"treatment"* or *"treating"* refers to an intervention (e.g. the administration of an agent to a subject) which cures, ameliorates or lessens the symptoms of a disease or removes (or lessens the impact of) its cause(s) (for example, the reduction in accumulation of pathological levels of lysosomal enzymes). In this case, the term is used synonymously with the term "therapy".

Additionally, the terms *"treatment"* or *"treating"* refers to an intervention (e.g. the administration of an agent to a subject) which prevents or delays the onset or progression of a disease or reduces (or eradicates) its incidence within a treated population. In this case, the term treatment is used synonymously with the term *"prophylaxis".*

As used herein, an *effective amount* or a *therapeutically effective amount* of an agent defines an amount that can be administered to a subject without excessive toxicity, irritation, allergic response, or other problem or complication, commensurate with a reasonable benefit/risk ratio, but one that is sufficient to provide the desired effect, e.g. the treatment or prophylaxis manifested by a permanent or temporary improvement in the subject's condition. The amount will vary from subject to subject, depending on the age and general condition of the individual, mode of administration and other factors. Thus, while it is not possible to specify an exact effective amount, those skilled in the art will be able to determine an appropriate *"effective"* amount in any individual case using routine experimentation and background general knowledge. A therapeutic result in this context includes eradication or lessening of symptoms, reduced pain or discomfort, prolonged survival, improved mobility and other markers of clinical improvement. A therapeutic result need not be a complete cure. Improvement may be observed in biological / molecular markers, clinical or observational improvements. In a preferred embodiment, the methods of the invention are applicable to humans, large racing animals (horses, camels, dogs), and domestic companion animals (cats and dogs).

In the context of treatment and effective amounts as defined above, the term *subject* (which is to be read to include *"individual", "animal", "patient"* or *"mammal'* where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, camels, bison, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human. As used herein, the term "equine" refers to mammals of the family *Equidae,* which includes horses, donkeys, asses, kiang and zebra.

*"Acelullar nerve graft"* refers to a section of nerve that has been treated to decellularise the nerve while maintaining the nerves structural and mechanical integrity and many of its ECM components, and that can be used in the treatment of nerve gap injury by bridging the severed ends of the nerves and providing a scaffold for migration of cells and generation of newly formed nerve tissue. The acellular nerve graft of the invention is particularly suitable for treatment of long nerve gap injury, especially long peripheral nerve gap injury.

*"Peripheral nerve"* refers to nerves that are outside the brain and spinal cord, and that connect the central nervous system to the limbs and organs.

*"Freezing and thawing"* refers to a step in the process of the invention in which a section of native nerve is frozen and then thawed with a view to decellularization of the section of nerve by rupturing cell membranes resulting in bursting of cells and cell lysis. In any embodiment, the section of nerve is frozen to -50°C, -60°C or -70°C or lower, typically to - 70°C to -90°C, typically about -75°C - 85°C, typically about -80°C and then thawed typically to room temperature, although it will be apparent to a person skilled in the art that other freezing and thawing temperatures may be employed to achieve decellularization.

*"Freeze-drying"* (also known as lyophilisation) is a process that comprises freezing the section of nerve, then lowering the pressure during drying phase and removing ice crystal formed in the nerve tissue by sublimation leaving pores in place of the ice crystals. The process typically involves a primary drying phase where the ice is forced to sublime, and a secondary drying phase in which unfrozen water molecules are removed. In one embodiment, the freeze-drying conditions comprise a freezing stage at about -30°C for about 1 hour, a primary drying stage at a temperature of about -10°C for about 24 hours, and a secondary drying stage at a temperature of about 20°C for about 5 hours. The method of the invention employs unidirectional freeze-drying, where the freeze-drying is configured to generate axially aligned ice crystals in the nerve tissue, resulting in axially aligned pores. In the present invention, the freeze-drying is generally *"longitudinal freeze drying",* which means that freeze-drying step is configured to generate longitudinal unidirectional pores in the section of nerve that are co-axial with longitudinal axis of the section of nerve. This may be achieved, for example, by arranging the section of nerve in the freeze-drying chamber in an upright position which promotes the growth of longitudinal ice crystals in the nerve tissue. In one embodiment, the section of nerve tissue is placed in a tube formed in an insulating block, and then placed on the cooling shelf of a freeze drier.

*"Average pore size"* as used herein is measured using the method described in Buckley et al. (Sridharan, R., Reilly, R.B. and Buckley C.T. Decellularized grafts with axially aligned channels for peripheral nerve regeneration. Journal of the Mechanical Behaviour of Biomedical Materials 41: 124-135, 2015).

*"Sulphated glycosaminoglycan"* or *"sGAG"* refers to long linear (unbranched) polysaccharides consisting of repeating disaccharide (double sugar) units. The repeating unit (except for keratan) consists of an amino sugar (N-acetylglucosamine or N-acetylgalactosamine) along with a uronic sugar (glucuronic acid or iduronic acid) or galactose. In one embodiment, the sGAG is a chondroitin sulphate, especially chondroitin-6-sulphate. In one embodiment, the invention provides an acellular nerve graft comprising exogenous sGAG and typically obtained by contacting the acellular nerve graft with a solution of sGAG, typically at elevated temperature. The term "elevated temperature" refers to a temperature that is greater than room temperature and lower than a temperature at which protein in the nerve tissue denatures, for example 25-40°C.

*"Allograft"* as used herein in the context of the use of the acellular nerve graft of the invention means that the nerve graft is prepared with a section of native nerve tissue from one subject, and used in treatment of a different subject. This is distinct from "autograft", where the donor of the native section of nerve and recipient of the prepared acellular graft are the same person. The use of an allograft provides many advantages over an autograft, including less hospital and operating theatre time for the patient, and a lowered risk of infection. The invention also relates to an acellular nerve graft of or obtaining by the invention, for use as an allograft or an autograft.

Decellularisation of nerve tissue with detergent and enzymatic decellularization agents is described in the literature, for example Sridharan et al described above. In the examples provided herein, the freeze dried section of nerve is decellularized with detergent and then with an enzyme. The detergent may be an ionic detergent. The detergent may be a bile acid, for example sodium deoxycholate. The detergent may be a nonionic detergent. The detergent may have a hydrophilic polyethylene oxide chain (e.g. on average it has 9.5 ethylene oxide units) and an aromatic hydrocarbon lipophilic or hydrophobic group. The detergent may be a Triton X-100. The nerves may be washed in a detergent solution, for example 1-10% w/v detergent. The nerve section may be washed in two detergents separately. The nerve section may be washed first in sodium deoxycholate and then in Triton X-100. The nerve may be washed at above room temperature, for example 30-40°C. The nerves may be incubated in buffer after the detergent wash(es). The nerves may be washed in one or more enzymatic agents, for example an agent that degrades nucleic acid such as a nuclease or benzonase. It will be appreciated that the choice of detergent and enzymatic agent may be varied provided that the agents are capable of decellularizing the section of nerve adequately. Likewise, the concentrations of the agents may be varied depending on the agent being employed, and the type of nerve being decellularized.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

### Development of an acellular porcine peripheral nerve graft with axially aligned pores

Peripheral nerves used for the fabrication of the decellularised allografts were harvested from porcine hind limbs (sciatic nerve branches). The excess fat and connective tissue from the nerves were trimmed away and washed in phosphate buffered saline solution (PBS) (Sigma Aldrich) to remove any excess blood from the tissue. The nerves were then cut into 5 cm segments and placed longitudinally in tubes lined with PBS soaked filter paper and kept at -80°C for storage.

The decellularisation process starts once the nerves were taken out of -80 storage and allowed to thaw. This physical process disrupts the cell membrane, burst cells and starts cell lysis. The thawed nerves were then placed in a freeze-drying polymer block (made out of an insulating polymer such as Ertalyte) and freeze dried longitudinally to create unidirectional pores.

The freeze-drying conditions consists of:
- 30°C (freezing stage) -1 hour
- 10 °C (primary drying stage) - 24 hours
20°C (secondary drying stage) - 5 hours

The unidirectionally freeze dried nerves were then subjected to chemical and enzymatic decellularisation process. All chemical processes and washing steps were carried out at 42°C under fast agitation (40 rpm). Enzymatic processes were carried out at 37°C under slow agitation (10 rpm) for two cycles, with each cycle lasting approximately 3 hours. The freeze dried nerves were first subjected to a chemical wash whereby the nerves were incubated in 4% Sodium Deoxycholate solution (Sigma) (pH 7.8-8.2) in distilled water for 24 hours and were then further incubated in 3% Triton X-100 solution (Fisher Scientific) (pH 7.2-7.6) in distilled water for 12-24 hours. The nerves were then washed three times in PBS (half an hour each time) and incubate overnight in PBS.

The nerves were then subjected to an enzymatic process whereby the nerves are incubated in a nuclease solution (50 mM tris buffer (Sigma), 1 mM MgCl2 (Fisher Scientific). 6H20, 1 U.mL -1 Benzonase (VWR); pH 7.5-7.7) made up in distilled water. The nerves were then washed three times in PBS (half an hour each time) and incubated overnight in PBS at 42°C. The nerves were then stored in 10mM phosphate (Sigma) and 50mM sodium (Sigma) solution in distilled water at 4 °C for short term storage (1-2 weeks).

### Characterisation of acellular peripheral nerve grafts of the invention

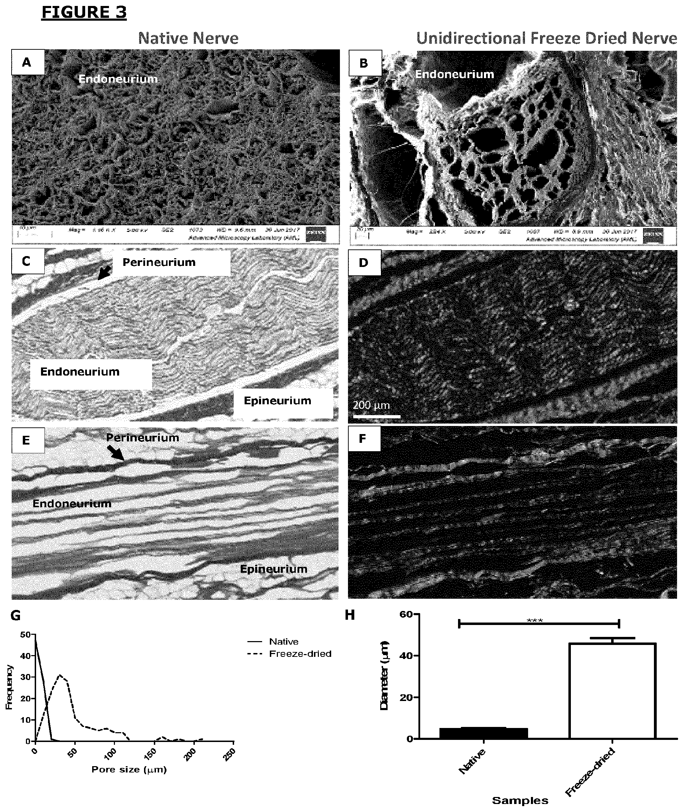

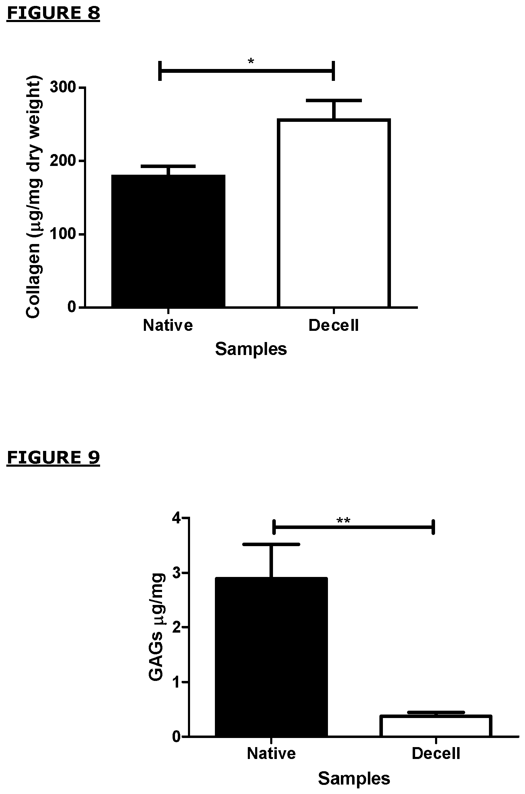

The acellular peripheral nerve sections were characterised by scanning electron microscopy (Fig. 3) and by immunohistological analysis (Figs, 5, 6 and 7)), and analysed for DNA content (Fig. 4) and collagen content (Fig. 8) according to the methods described in Buckley et al..

### Mechanical evaluation of native and acellular porcine nerves

As indicated in Table 1 below, mechanical analysis revealed that the decellularisation process does not affect the mechanical properties of the nerves. The table reveals no significant changes in the UTS, strain or Young Modulus in when compared to their native nerve counterparts.

**TABLE 1**

| Tensile Testing | | | |
|---|---|---|---|
| Samples | Ultimate Tensile Stress UTS (MPa) | Strain % | Young's Modulus (MPa) |
| Native porcine nerve (n = 5) | 0.37 ± 0.067 | 64.02 ± 20.92 | 0.01336 ± 0.0034 |
| Acellular porcine nerves (n = 8) | 0.44 ± 0.1260 | 62.00 ± 12.59 | 0.01022 ± 0.0022 |

### Unidirectional freeze-drying has beneficial effect on enhancing VEGF release compared to standard freeze-drying protocol

Nerves were cut up into 1 x1 cm segments, placed in 24 well plates and primed with 10% DMEM overnight. The media was then taken off and dorsal root ganglions (DRG's) were added on top on top. 1.5 mL of 10% media was added to each sample (enough to cover the samples but careful not wash away the DRG's) and transferred to the incubator. Media from each sample was taken every 2 days and stored in labelled test tubes in the -20 freezer. Samples were analysed using ELISA (enzyme linked immunosorbent assay).

Three experimental groups were evaluated
1. Unidirectionally freeze dried decelled nerves
2. Normal freeze dried decelled nerves
3. Decelled nerves

While lower than Decelled group, unidirectional freeze-drying has beneficial effect on enhancing VEGF release compared to standard freeze-drying protocol. This has implications on subsequent bioactivity.

### Introduction of chondroitin-6-sulphate into acellular nerve tissue

Unidirectional freeze dried acellular nerves are prepared as described above. The nerves are then incubated in a 50µg/mL GAG solution made up to 50 mL in PBS overnight under slow agitation (10 rpm) at 37°C. Sulphated GAG content in acellular nerve tissue after the addition of chondroitin-6-sulphate is shown in Figure 10.

### Sterilisation and biocompatibility assessment:

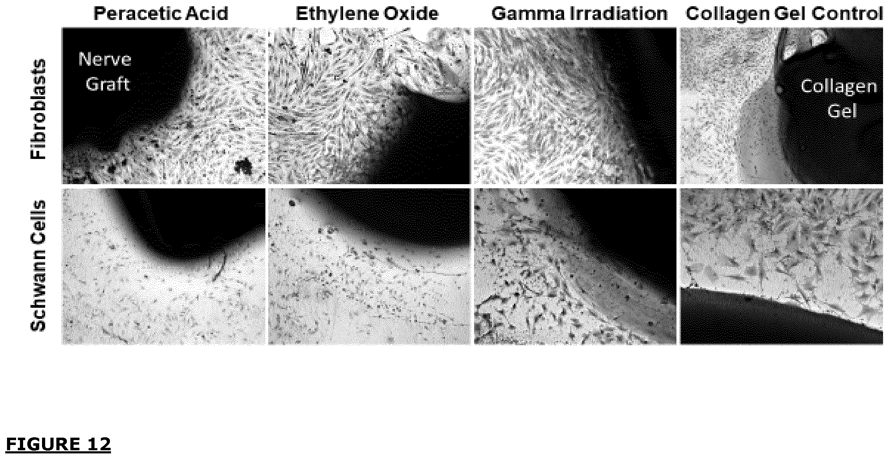

Decellularised nerve segments were sterilised using either peracetic acid, ethylene oxide or gamma irradiation. In vitro cell compatibility assays were performed with these sterilised decellularised nerve segments using fibroblasts and Schwann cells, with a collagen gel serving as a control [Figure 12].

### Assessment of biomechanical tensile properties:

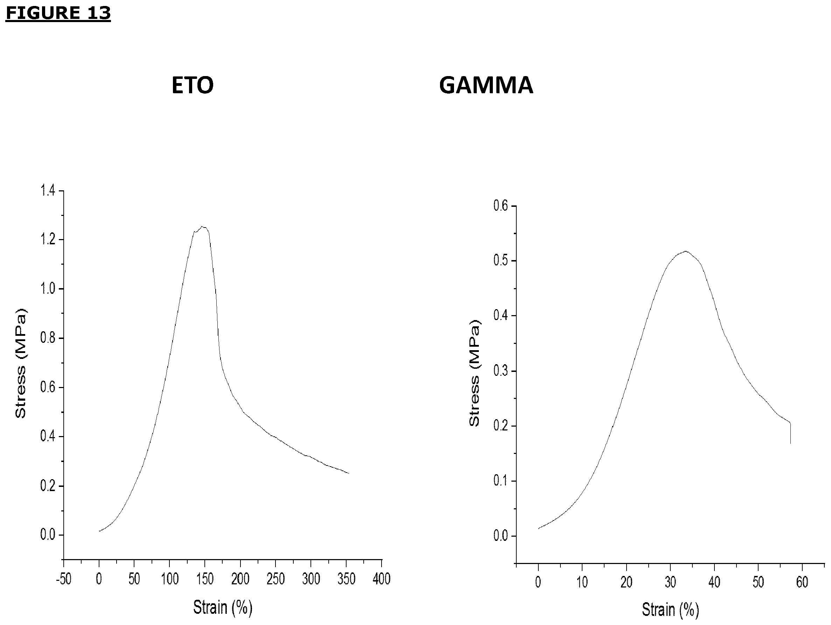

Uniaxial tensile tests were performed using a Zwick tensile testing machine (Zwick Z005, Roell, Germany) [Figure 13 and Table 2].

**TABLE 2**

| **Tensile Testing** | | | |
|---|---|---|---|
| **Samples** | **Ultimate tensile strength UTS (MPa)** | **Strain %** | **Young's Modulus (MPa)** |
| Native porcine nerve (n = 7) | 0.49 ± 0.1050 | 68.06 ± 20.92 | 0.010 ± 0.003887 |
| ETO porcine nerve (n = 9) | 0.92 ± 0.2865 * | 76.86 ± 12.59 | 0.021 ± 0.002241** |
| GAMMA porcine nerve (n = 7) | 0.49 ± 0.17 | 52.30 ± 12.59 | 0.015 ± 0.002241 |

### In Vivo Rat Sciatic Nerve Defect Model:

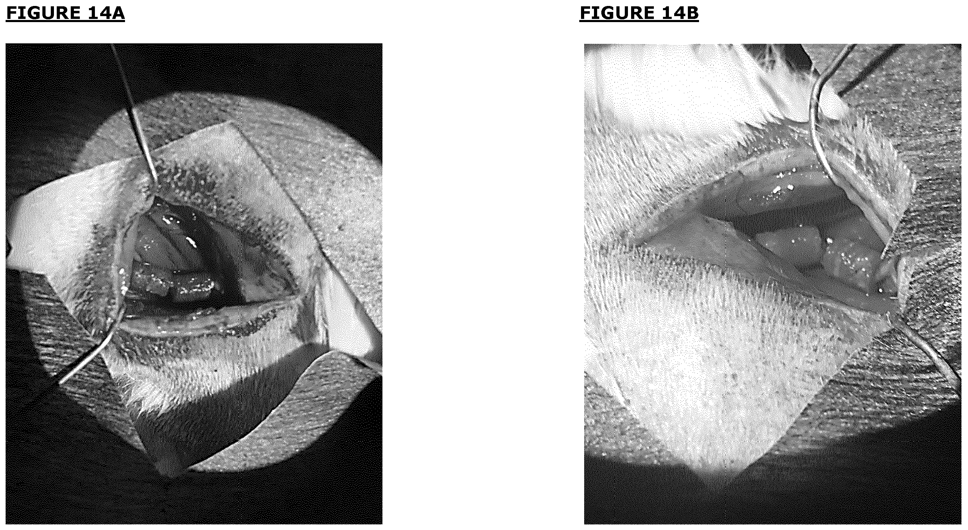

Decellularised nerve segments were evaluated in vivo in a 15 mm sciatic nerve defect in adult Lewis rats, 294 ± 19 g (Envigo, Blackthorn, United Kingdom), with inverted autografts serving as controls [Figure 14]. After 8 weeks post-implantation, animals were humanely euthanized using an overdose of anaesthetic followed by cervical dislocation.

### Functional Recovery Assessment:

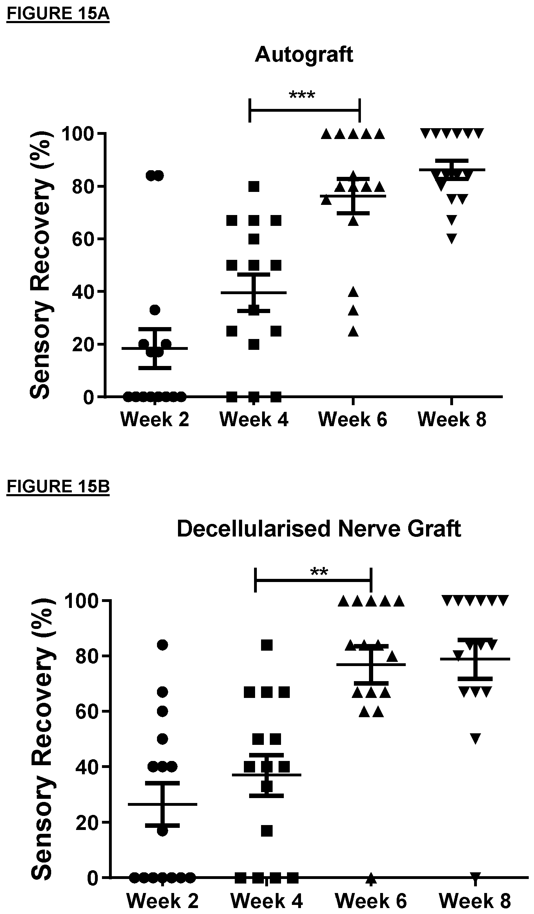

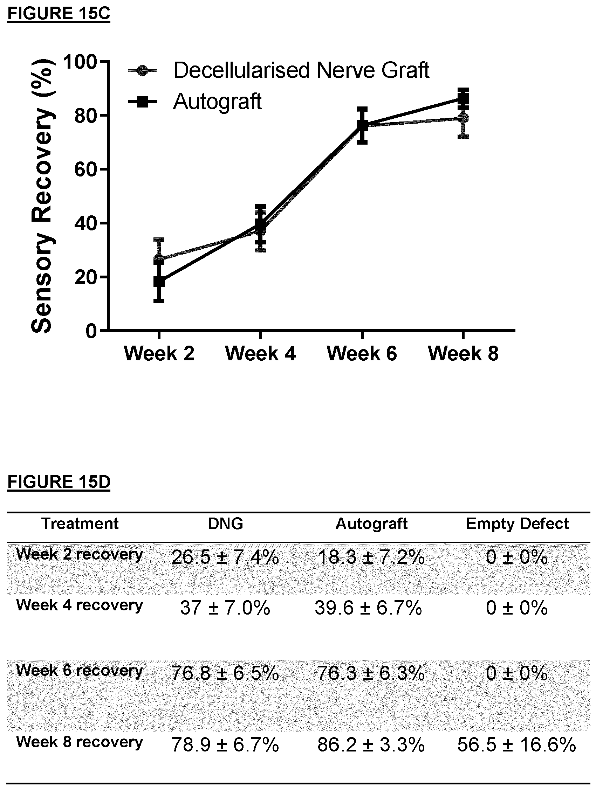

Functional improvement in the operated limb was assessed every 2 weeks using a response to electrical stimulation (0.1 mA to 0.6 mA) [Figure 15].

### Electrophysiology:

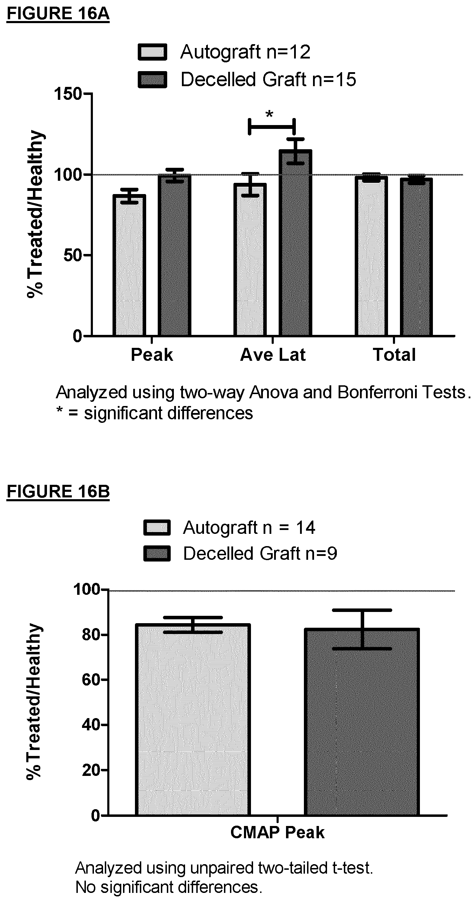

Compound nerve action potential (CNAP) and compound muscle action potential (CMAP) was determined (100,000 samples per second with a 10 kHZ lowpass filter and 25mV gain) by stimulating the nerve using a concentric needle electrode (stimulus intensity of 1mA) and recording with a second electrode in the distal end (CNAP) or midbelly of the gastrocnemius muscle (CMAP). Both CNAP and CMAP are expressed as a percentage of the non-operated contralateral limb recordings [Figure 16].

### Wet muscle weight:

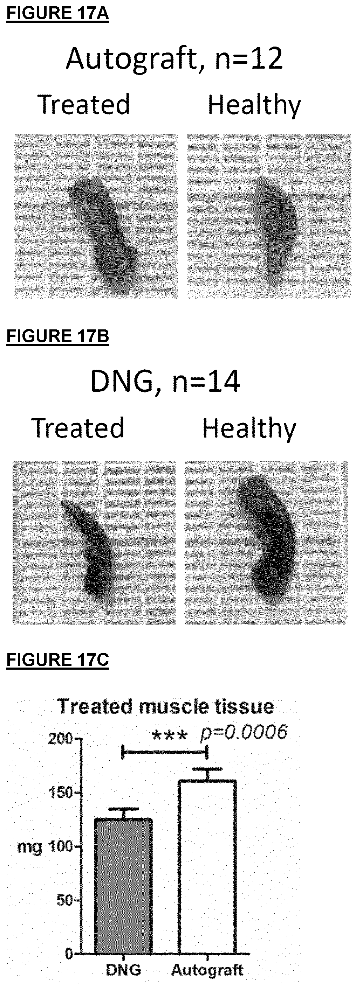

The tibialis anterior muscle was harvested after 8 weeks post-surgery, washed once in PBS solution, dabbed on blotting paper to remove excess liquid and weighed [Figure 17].

### Histomorphometry and Immunofluorescence Staining:

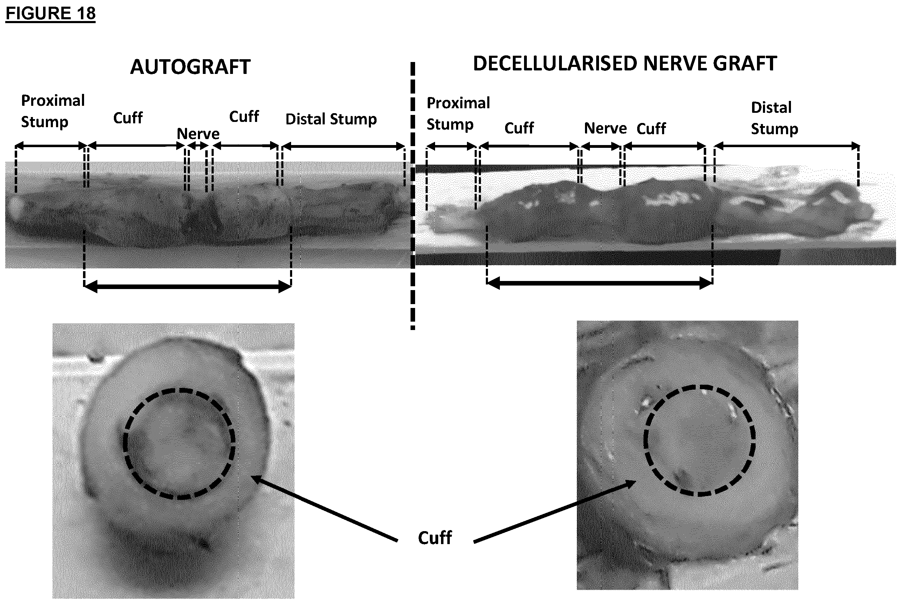

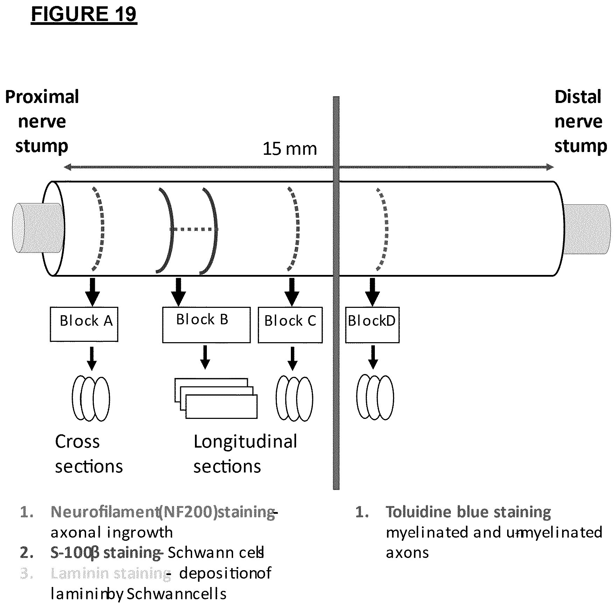

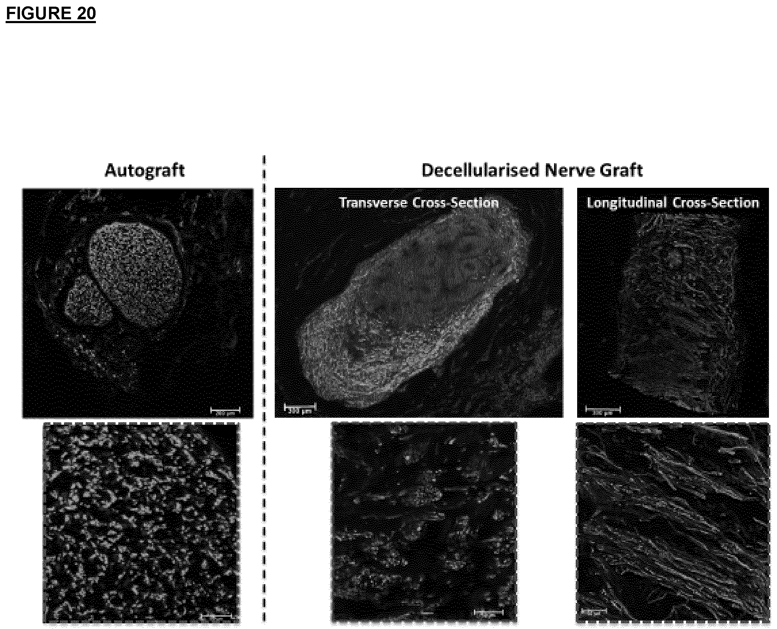

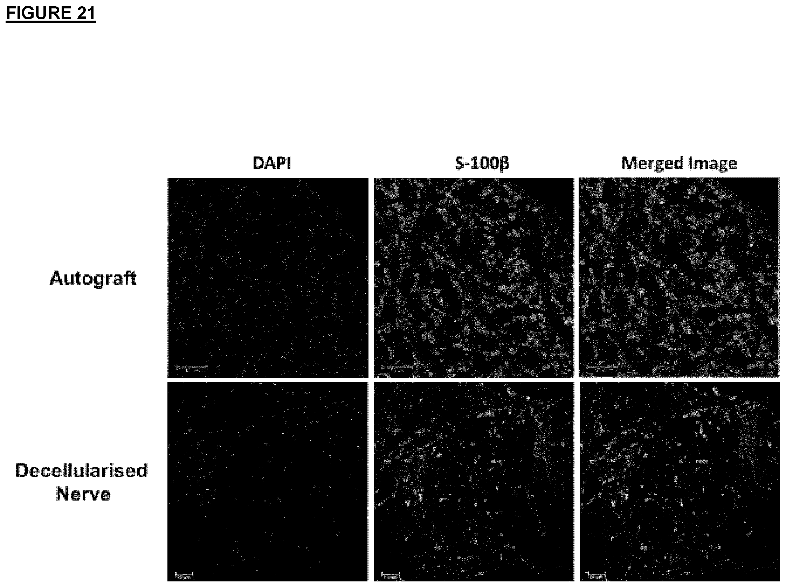

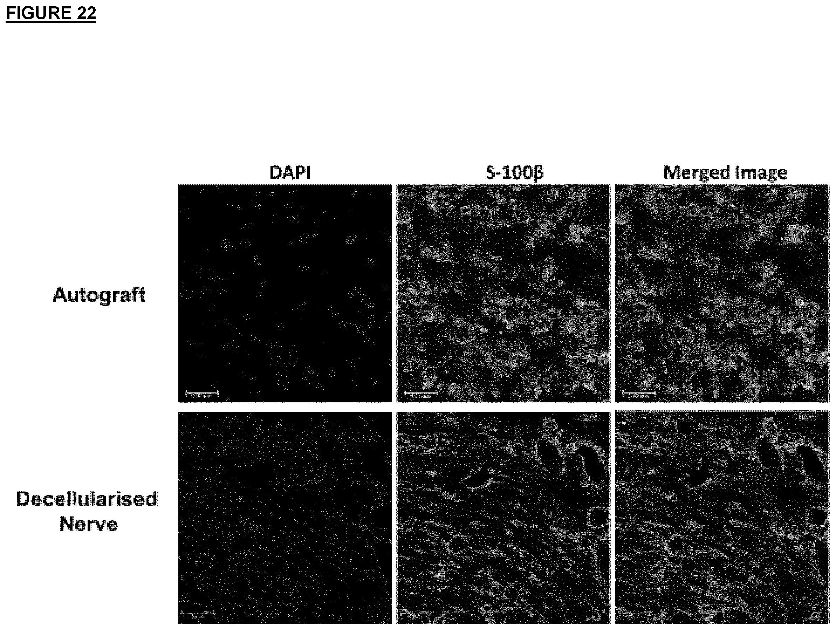

Excised decellularised [Figure 18] nerve segments and autograft controls were fixed using 4% paraformaldehyde. Samples were washed in phosphate buffered saline (PBS) and divided into blocks [Figure 19] for toluidine blue staining (one block in proximal section and another block in distal section) to assess the nerve histomorphometry and immunofluorescent staining (one proximal block, one middle-graft block and one distal block) to assess the presence of axons, Schwann cells and extracellular matrix components [Figures 20, 21, 22].. Staining was performed using standard protocols available in the scientific literature.

## Claims

1. A method of producing an acellular peripheral nerve graft comprising the steps of:
providing a section of peripheral nerve;
primary treatment of the section of peripheral nerve comprising freezing and then thawing the section of peripheral nerve;
freeze-drying the thawed section of peripheral nerve longitudinally and unidirectionally to introduce longitudinal pores into the section of peripheral nerve having an average pore size of at least 40 µm; and
decellularization of the section of peripheral nerve comprising contacting the freeze-dried section of peripheral nerve with detergent and enzymatic decellularization agents to provide the acellular peripheral nerve graft having a DNA content of less than 70 ng/mg.

2. A method according Claim 1, in which the acellular peripheral nerve graft has a DNA content of less than 60 ng/mg.

3. A method according to Claim 1 or 2, in which the freeze-drying step comprises freeze-drying the thawed section of peripheral nerve in an upright position in the freeze-drying chamber.

4. A method according to Claim 3, in which the thawed section of peripheral nerve is placed into an upright tube formed in an insulating block and freeze-dried in an upright position within the insulating block.

5. A method according to any preceding Claim, including a further step of contacting the acellular peripheral nerve graft with a solution of chondroitin-6-sulphate at a temperature that is greater than room temperature and lower than a temperature at which protein in the nerve tissue denatures.

6. A method according to Claim 5, in which the of contacting the acellular peripheral nerve graft with a solution of chondroitin-6-sulphate is at 25-40°C.

7. A method according to any preceding Claim, in which the section of peripheral nerve has a length of at least 50 mm.

8. A method according to any preceding Claim in which the primary treatment step comprises freezing the section of peripheral nerve to -70°C to -90°C.

9. A method according to any preceding Claim, in which the freeze-drying conditions comprise a freezing stage, a primary drying stage at a temperature of less than 0°C for at least 12 hours, and a secondary drying stage of at a temperature of more than 10°C for at least 2 hours.

10. A method according to any preceding Claim, in which the freeze-drying conditions comprise a freezing stage at about -30°C for about 1 hour, a primary drying stage at a temperature of about -10°C for about 24 hours, and a secondary drying stage at a temperature of about 20°C for about 5 hours.

11. An acellular peripheral nerve graft formed by a method of any of Claims 1 to 10 having longitudinal unidirectional pores with an average pore size of at least 40 µm and a DNA content of less than 70 ng/mg resulting from longitudinally and unidirectionally freeze-drying a thawed section of a peripheral nerve prior to decellularization of the section.

12. An acellular peripheral nerve graft according to Claim 11, having a native nerve structure with retention of nerve fascicles and connective tissue layers, endoneurium, perineuriem and epineurium.

13. An acellular peripheral nerve graft according to any of Claims 11 to 12 having an average pore size of at least 60 µm.

14. An acellular peripheral nerve graft according to Claim 11, formed by a method of any of Claims 5 to 9, having a sulphated glycosaminoglycan (sGAG) content of greater than 0.21 µg/mg.

## Patentansprüche

1. Verfahren zur Herstellung eines azellulären peripheren Nerventransplantats, das die folgenden Schritte umfasst:
Bereitstellen eines Abschnitts von peripherem Nerv;
Primärbehandlung des Abschnitts von peripherem Nerv, die das Gefrieren und dann Auftauen des Abschnitts von peripherem Nerv umfasst;
Gefriertrocknen des aufgetauten Abschnitts von peripherem Nerv in Längsrichtung und unidirektional, um Längsporen in den Abschnitt von peripherem Nerv einzuführen, die eine durchschnittliche Porengröße von mindestens 40 µm aufweisen; und
Dezellularisierung des Abschnitts von peripherem Nerv, die das Inkontaktbringen des gefriergetrockneten Abschnitts von peripherem Nerv mit Detergens und enzymatischen Dezellularisierungsmitteln umfasst, um das azelluläre periphere Nerventransplantat bereitzustellen, das einen DNA-Gehalt von weniger als 70 ng/mg aufweist.

2. Verfahren nach Anspruch 1, in dem das azelluläre periphere Nerventransplantat einen DNA-Gehalt von weniger als 60 ng/mg aufweist.

3. Verfahren nach Anspruch 1 oder 2, in dem der Gefriertrocknungsschritt das Gefriertrocknen des aufgetauten Abschnitts von peripherem Nerv in einer aufrechten Position in der Gefriertrocknungskammer umfasst.

4. Verfahren nach Anspruch 3, in dem der aufgetaute Abschnitt von peripherem Nerv in einem aufrechten Röhrchen platziert wird, das in einem isolierenden Block ausgebildet ist, und in einer aufrechten Position innerhalb des isolierenden Blocks gefriergetrocknet wird.

5. Verfahren nach einem vorstehenden Anspruch, das einen weiteren Schritt des Inkontaktbringens des azellulären peripheren Nerventransplantats mit einer Lösung von Chondroitin-6-sulfat bei einer Temperatur einschließt, die höher ist als Raumtemperatur und niedriger als eine Temperatur ist, bei der Protein in dem Nervengewebe denaturiert.

6. Verfahren nach Anspruch 5, in dem das Inkontaktbringen des azellulären peripheren Nerventransplantats mit einer Lösung von Chondroitin-6-sulfat bei 25-40 °C erfolgt.

7. Verfahren nach einem vorstehenden Anspruch, in dem der Abschnitt von peripherem Nerv eine Länge von mindestens 50 mm aufweist.

8. Verfahren nach einem vorstehenden Anspruch, in dem der Primärbehandlungsschritt das Gefrieren des Abschnitts von peripherem Nerv bei -70°C bis -90 °C umfasst.

9. Verfahren nach einem vorstehenden Anspruch, in dem die Gefriertrocknungsbedingungen eine Gefrierphase, eine primäre Trocknungsphase bei einer Temperatur von niedriger als 0 °C für mindestens 12 Stunden und eine sekundäre Trocknungsphase bei einer Temperatur von höher als 10 °C für mindestens 2 Stunden umfassen.

10. Verfahren nach einem vorstehenden Anspruch, in dem die Gefriertrocknungsbedingungen eine Gefrierphase bei etwa -30 °C für etwa 1 Stunde, eine primäre Trocknungsphase bei einer Temperatur von etwa -10 °C für etwa 24 Stunden und eine sekundäre Trocknungsphase bei einer Temperatur von etwa 20 °C für etwa 5 Stunden umfassen.

11. Azelluläres peripheres Nerventransplantat, das durch ein Verfahren nach einem der Ansprüche 1 bis 10 gebildet ist, längsgerichtete, unidirektionale Poren mit einer durchschnittlichen Porengröße von mindestens 40 µm und einen DNA-Gehalt von weniger als 70 ng/mg aufweist, die durch längsgerichtetes und unidirektionales Gefriertrocknen eines aufgetauten Abschnitts eines peripheren Nervs vor der Dezellularisierung des Abschnitts entstehen.

12. Azelluläres peripheres Nerventransplantat nach Anspruch 11, das eine native Nervenstruktur mit Bewahrung von Nervenfaszikeln und Bindegewebeschichten, Endoneurium, Perineurium und Epineurium, aufweist.

13. Azelluläres peripheres Nerventransplantat nach einem der Ansprüche 11 bis 12, das eine durchschnittliche Porengröße von mindestens 60 µm aufweist.

14. Azelluläres peripheres Nerventransplantat nach Anspruch 11, das durch ein Verfahren nach einem der Ansprüche 5 bis 9 gebildet ist, einen Gehalt von sulfatiertem Glykosaminoglykan (sGAG) von größer als 0,21 µg/mg aufweist.

## Revendications

1. Procédé de réalisation d'une greffe de nerf périphérique acellulaire comprenant les étapes de :
la fourniture d'une section de nerf périphérique ;
le traitement primaire de la section de nerf périphérique comprenant une congélation puis une décongélation de la section de nerf périphérique ;
la lyophilisation de la section de nerf périphérique décongelée longitudinalement et unidirectionnellement pour introduire des pores longitudinaux dans la section de nerf périphérique présentant une taille de pore moyenne d'au moins 40 µm ; et
la décellularisation de la section de nerf périphérique comprenant la mise en contact de la section de nerf périphérique lyophilisée avec un détergent et des agents de décellularisation enzymatiques pour fournir la greffe de nerf périphérique acellulaire présentant une teneur en ADN inférieure à 70 ng/mg.

2. Procédé selon la revendication 1, dans lequel la greffe de nerf périphérique acellulaire présente une teneur en ADN inférieure à 60 ng/mg.

3. Procédé selon la revendication 1 ou la revendication 2, dans lequel l'étape de lyophilisation comprend la lyophilisation de la section de nerf périphérique décongelée en position verticale dans la chambre de lyophilisation.

4. Procédé selon la revendication 3, dans lequel la section de nerf périphérique décongelée est placée dans un tube vertical formé dans un bloc isolant et lyophilisée en position verticale à l'intérieur du bloc isolant.

5. Procédé selon une quelconque revendication précédente, incluant une étape supplémentaire de mise en contact de la greffe de nerf périphérique acellulaire avec une solution de chondroïtine-6-sulfate à une température supérieure à la température ambiante et inférieure à une température à laquelle la protéine dans le tissu nerveux est dénaturée.

6. Procédé selon la revendication 5, dans lequel la mise en contact de la greffe de nerf périphérique acellulaire avec une solution de chondroïtine-6-sulfate est à 25-40 °C.

7. Procédé selon une quelconque revendication précédente, dans lequel la section de nerf périphérique présente une longueur d'au moins 50 mm.

8. Procédé selon une quelconque revendication précédente dans lequel l'étape de traitement primaire comprend la congélation de la section de nerf périphérique de -70 °C à -90 °C.

9. Procédé selon une quelconque revendication précédente, dans lequel les conditions de lyophilisation comprennent une étape de congélation, une étape de séchage primaire à une température inférieure à 0 °C pendant au moins 12 heures, et une étape de séchage secondaire à une température supérieure à 10 °C pendant au moins 2 heures.

10. Procédé selon une quelconque revendication précédente, dans lequel les conditions de lyophilisation comprennent une étape de congélation à environ -30 °C pendant environ 1 heure, une étape de séchage primaire à une température d'environ -10 °C pendant environ 24 heures, et une étape de séchage secondaire à une température d'environ 20 °C pendant environ 5 heures.

11. Greffe de nerf périphérique acellulaire formée par un procédé selon l'une quelconque des revendications 1 à 10 présentant des pores unidirectionnels longitudinaux d'une taille de pore moyenne d'au moins 40 µm et une teneur en ADN inférieure à 70 ng/mg résultant de la lyophilisation longitudinale et unidirectionnelle d'une section de nerf périphérique décongelée avant la décellularisation de la section.

12. Greffe de nerf périphérique acellulaire selon la revendication 11, présentant une structure nerveuse native avec rétention de fascicules nerveux et de couches de tissu conjonctif, d'endonèvre, de périnèvre et d'épinèvre.

13. Greffe de nerf périphérique acellulaire selon l'une quelconque des revendications 11 et 12 présentant une taille de pore moyenne d'au moins 60 µm.

14. Greffe de nerf périphérique acellulaire selon la revendication 11, formée par un procédé selon l'une quelconque des revendications 5 à 9, présentant une teneur en glycosaminoglycanes sulfatés (sGAG) supérieure à 0,21 µg/mg.
